# EUROPEAN PATENT APPLICATION

(11) **EP 4 663 129 A1**
(43) Date of publication of application: **17.12.2025**
(21) Application number: 25178694.3
(22) Date of filing: 26.05.2025
(51) Int. Cl.: A61B 6/04, A61B 6/50, A61B 6/51

(54) **SUBJECT ALIGNMENT DEVICE WITH DETACHABLE GUIDE UNIT AND X-RAY RADIOGRAPHY APPARATUS INCLUDING SAME**

(30) Priority: 13.06.2024 KR 20240077220
(71) Applicant: Vatech Co., Ltd., Hwaseong-si, Gyeonggi-do 18449 (KR); Vatech Ewoo Holdings Co., Ltd., Hwaseong-si, Gyeonggi-do 18449 (KR)
(72) Inventor: Choi, Sung Il, 18449 Hwaseong-si, Gyeonggi-do (KR); Jang, Won Park, 18449 Hwaseong-si, Gyeonggi-do (KR)
(74) Representative: Lorenz & Kollegen

(57) **Abstract**

Proposed is a subject alignment device of an X-ray radiography apparatus for obtaining at least two types of X-ray images of a subject. The subject alignment device includes a guide part configured to guide the subject into a radiography posture for obtaining the X-ray images, and guide units configured cc coupled to the guide part according to the types of the X-ray images, and configured to respectively contact different positions of the subject.

## Description

### CROSS REFERENCE TO RELATED APPLICATION

The present application claims priority to Korean Patent Application No. 10-2024-0077220, filed June 13, 2024, the entire contents of which is incorporated herein for all purposes by this reference.

### BACKGROUND OF THE INVENTION

### Field of the Invention

The present disclosure relates to a subject alignment device with a detachable guide unit, and an X-ray radiography apparatus including the subject alignment device.

### Description of the Related Art

An X-ray radiography apparatus is provided with a subject alignment device for aligning a subject to a proper position and posture. Since required conditions vary depending on the types of X-ray images, the structures and functions of subject alignment devices used differ depending on the types of X-ray images.

For example, in the case of a dental X-ray radiography apparatus, in order to maintain a vertical posture of the head without obstructing the head during CT radiography, temple supports that press and fix the left and right temples or a guide frame that comes into close contact with the face along its edge maintains the left-right horizontal alignment of the head during cephalometric radiography, and ear rods inserted into the left and right ears may be used so that the left-right horizontal alignment of the head is checked on the X-ray image.

Herein, since the functions and structures of subject alignment devices used in various types of X-ray radiography are different from other, there is a problem in that when the radiography method is changed, the entire radiography apparatus needs to be replaced or the subject's radiography position needs to be changed.

The technology behind the present disclosure is disclosed in Korean Patent No. 10-1659177.

The foregoing is intended merely to aid in the understanding of the background of the present disclosure, and is not intended to mean that the present disclosure falls within the purview of the related art that is already known to those skilled in the art.

### SUMMARY OF THE INVENTION

The present disclosure is directed to providing a subject alignment device with a detachable guide unit for aligning a subject's posture, and an X-ray radiography apparatus including the subject alignment device.

However, technical objectives that the embodiment of the present disclosure is intended to achieve are not limited to the above-described technical objectives, and there may be other technical objectives.

As a technical means for realizing the technical objective of the present disclosure, according to a first aspect of the present disclosure, there is provided a subject alignment device of an X-ray radiography apparatus for obtaining at least two types of X-ray images of a subject, the subject alignment device including: a guide part configured to guide the subject into a radiography posture for obtaining the X-ray images; and guide units configured to be replaceably and detachably coupled to the guide part according to the types of the X-ray images, and configured to respectively contact different positions of the subject.

In addition, as a technical means for realizing the technical objective of the present disclosure, there is provided an X-ray radiography apparatus including a subject alignment device according to the first aspect of the present disclosure.

The above-mentioned solutions are merely exemplary and should not be construed as limiting the present disclosure. In addition to the above-described exemplary embodiment, additional embodiments may exist in the drawings and detailed description of the disclosure.

According to the present disclosure, by providing a common guide part and a guide unit replaceably and detachably coupled to the guide part depending on the type of X-ray radiography, the subject alignment device suitable for the desired type of an X-ray radiography image can be used only with the detachable guide unit without changing the entire X-ray radiography apparatus or the subject's radiography position, thereby achieving convenient and rapid X-ray radiography.

However, effects achieved by the present disclosure are not limited to the above-described effects, and there may be other effects.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a schematic diagram illustrating a subject alignment device according to an embodiment of the present disclosure;
FIG. 2 is a schematic diagram illustrating a first guide unit of a subject alignment device according to an embodiment of the present disclosure;
FIG. 3 is a schematic diagram illustrating a second guide unit of a subject alignment device according to an embodiment of the present disclosure;
FIG. 4 is a schematic diagram illustrating a modification of a first guide unit of a subject alignment device according to an embodiment of the present disclosure; and
FIG. 5 is a schematic diagram illustrating a third guide unit of a subject alignment device according to an embodiment of the present disclosure.

### DETAILED DESCRIPTION OF THE INVENTION

Hereinbelow, an exemplary embodiment of the present disclosure will be described in detail with reference to the accompanying drawings such that the present disclosure can be easily embodied by those skilled in the art to which this present disclosure belongs. However, the present disclosure may be embodied in various different forms and should not be limited to the embodiment set forth herein. Further, in order to clearly explain the present disclosure, portions that are not related to the present disclosure are omitted in the drawings, and like reference numerals designate like elements throughout the specification.

Throughout the specification of the present disclosure, when a part is referred to as being "connected" to another part, it includes not only being "directly connected", but also being "electrically connected" with an intervening device therebetween.

Throughout the specification, when a member is said to be "on", "at an upper portion of", "on top of", "under", "at a lower portion of", "at the bottom of" another member, this includes not only when the two members are in contact, but also when there is an intervening member between the two members.

Throughout the specification, when a part "includes" an element, it is noted that it further includes other elements, but does not exclude other elements, unless specifically stated otherwise.

The present disclosure relates to a subject alignment device (hereinafter, referred to as "the device") 100 with a detachable guide unit, and an X-ray radiography apparatus including the device. The X-ray radiography apparatus may be, for example, an apparatus for CT radiography or an apparatus for cephalometric X-ray radiography, but is not limited thereto.

The device 100 may be a device for aligning the subject's posture and position to ensure accurate X-ray radiography. For example, in the case of a subject alignment device included in a dental X-ray radiography apparatus, the device may be configured to fix the position, angle, and posture of the head of a target, such as a person, being radiographed in order to accurately radiograph the teeth of the person.

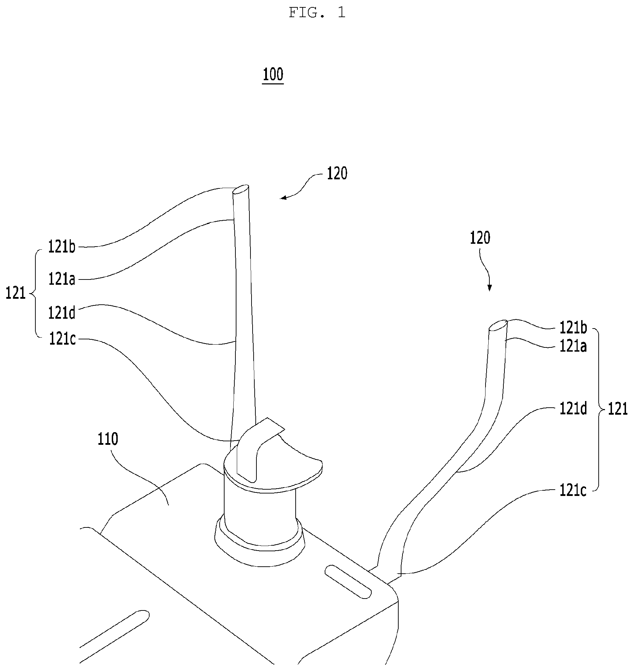

FIG. 1 is a schematic diagram illustrating a subject alignment device according to an embodiment of the present disclosure.

Referring to FIG. 1, the device 100 may include a base 110. The base 110 may be to fix a member that comes into close contact directly with the subject to fix or align the subject's posture. For example, the base 110 may be installed to be fixed to a floor, a table, or a wall and may align the subject. In addition, the base 110 may be a part of the X-ray radiography apparatus. For example, the base 110 may be fixed to a floor, a table, or a wall, and a radiography part for X-ray radiography and the device 100 for aligning the subject may be placed with respect to the base to constitute the X-ray radiography apparatus. However, no limitation thereto is imposed.

In addition, referring to FIG. 1, the device 100 may include a pair of bar-shaped guide parts 120 installed on the base 110 to extend upward from the base 110 and placed spaced apart from each other in the left-right direction. Herein, the left-right direction may correspond to the 4 o'clock-10 o'clock direction in FIG. 1. That is, in FIG. 1, the 4 o'clock direction may correspond to the left direction, the 10 o'clock direction may correspond to the right direction. In addition, the 2 o'clock direction may correspond to the rearward direction, the 8 o'clock direction may correspond to the forward direction, the 12 o'clock direction may correspond to the upward direction, and the 6 o'clock direction may correspond to the downward direction.

Referring to FIG. 1, each of the guide parts 120 in the pair may include a bar member 121 and a guide structure 122 provided at an intermediate portion of the bar member 121. The bar member 121 may be a bar-shaped member having a length extending upward (in the 12 o'clock direction in FIG. 1) from the base 110. In addition, the bar member 121 may be a member for supporting or fixing the guide unit. The bar member 121 may be a member having a straight shape, or may be a member having a curved shape.

The guide structure 122 may be provided to guide an attachment/detachment position of the guide unit for aligning the subject's position or posture or both. That is, the guide unit may be provided to come into close contact with the subject so as to align the subject's position or posture or both, and the guide structure 122 may be provided at an intermediate portion of the bar member 121 so that the guide unit is detachable from the bar member 121. The guide structure 122 may be formed by deforming a portion of the bar member 121, or may be formed by placing a separate member.

The bar member 121 may include an upper member 121a in which the guide structure 122 is provided at an intermediate portion. The upper member 121a may be a portion of an upper part of the bar member 121. For example, referring to FIG. 1, a straight-shaped portion at the upper part of the bar member 121 may correspond to the upper member 121a, but is not limited thereto.

Referring to FIG. 1, the upper member 121a may have an upper end part 121b that is detachably coupled to at least one of a plurality of types of guide units, which vary according to the types of images to be obtained through X-ray radiography. The upper end part 121b may be, for example, positioned at the upper distal end portion of the bar member 121. The upper end part 121b may be formed by deforming a portion of the bar member 121 or may be formed by placing a separate member so as to be detachably coupled to at least one of the plurality of types of guide units. Alternatively, without deformation of the bar member 121 or placement of a separate member, a portion of the bar member 121 itself may correspond to the upper end part 121b.

The guide structure 122 and the upper end part 121b may have a spacing therebetween and respective shapes set such that any one of the plurality of types of guide units is mounted at each of the guide parts 120 in the pair, using the guide structure 122 or the upper end part 121b or both. That is, the guide structure 122 and the upper end part 121b may be provided with appropriate shapes or may be provided to have appropriate positions and a spacing therebetween such that any one of the plurality of types of guide units is mounted to each of the guide parts 120 in the pair, thus enabling the various types of guide units to be detachable from the pair of guide parts 120. Accordingly, even when a plurality of types of X-ray radiography are performed, the most suitable guide unit for each type of X-ray radiography is mounted, thereby enabling effective and rapid X-ray radiography.

Referring to FIG. 1, the bar member 121 may further include: a mounting member 121c mounted to the base 110; and an inclined member 121d that obliquely extends upward and and rearward from the mounting member 121c and is connected to the lower end of the upper member 121a. For example, the mounting member 121c may be a member that connects the pair of guide parts 120 to the base 110 such that the pair of guide parts 120 are mounted to the base 110. For example, the mounting member 121c may be mounted to the base 110 by screw-type coupling, but is not limited thereto.

The base 110 may be provided with a left-right width narrower than the facial width of a typical adult. For example, the base 110 may be provided with a left-right width narrower than the average facial width of an adult, but is not limited thereto.

Referring to FIG. 1, the inclined members 121d of the guide parts 120 in the pair may extend having an inclination such that the spacing therebetween in the left-right direction gradually increases toward the rear. For example, the inclined members 121d may be provided to have an inclination in a direction rearward from the device 100 and away from each other. That is, the right bar of the pair of guide parts 120 may obliquely extend upward in a rearward-rightward direction, and the the left bar of the pair of guide parts 120 may obliquely extend upward in a rearward-leftward direction.

The base 110 may be provided such that the mounting members 121c of the guide parts 120 in the pair are mounted to enable the spacing therebetween in the left-right direction to be adjustable within a preset width range. Through this, even when X-ray radiography is performed using the device 100 for various individuals having different physical characteristics, the spacing between the guide parts 120 in the pair may be adjusted so as to be most suitable for the characteristics of a target being radiographed.

Herein, the preset width range may be set such that the spacing between the upper members 121a connected to the upper ends of the inclined members 121d of the guide parts 120 in the pair is adjustable within a range including the facial width of a typical adult. In other words, the spacing between the upper members 121a may be adjustable to accommodate the facial width of a typical adult.

For example, at least one slot extending in the left-right direction may be formed at the upper surface or the rear surface of the base 110, and at least one of the mounting members 121c of the guide parts 120 in the pair may be mounted to the slot such that the spacing therebetween in the left-right direction is adjustable. That is, the pair of guide parts 120 is mounted to the slot formed at the base 110 in such a manner that each of the guide parts 120 in the pair is movable in the left-right direction with respect to the base 110 and the slot, thereby enabling the spacing between the guide parts 120 in the pair to be adjustable. However, the coupling structure or mounting structure for adjusting the spacing between the guide parts 120 in the pair is not limited to the above-described slot, and other types of coupling structures, such as a rail structure, may also be applied.

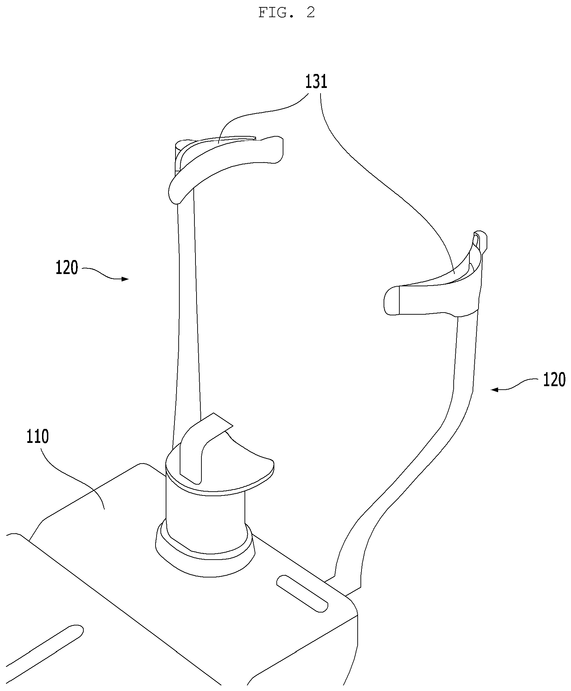

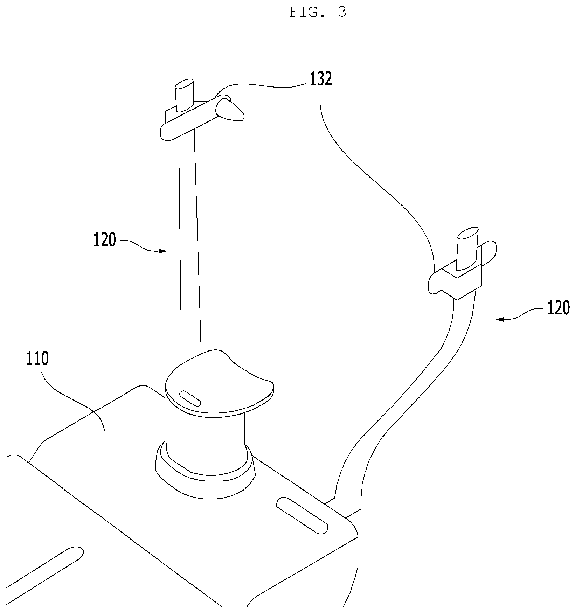

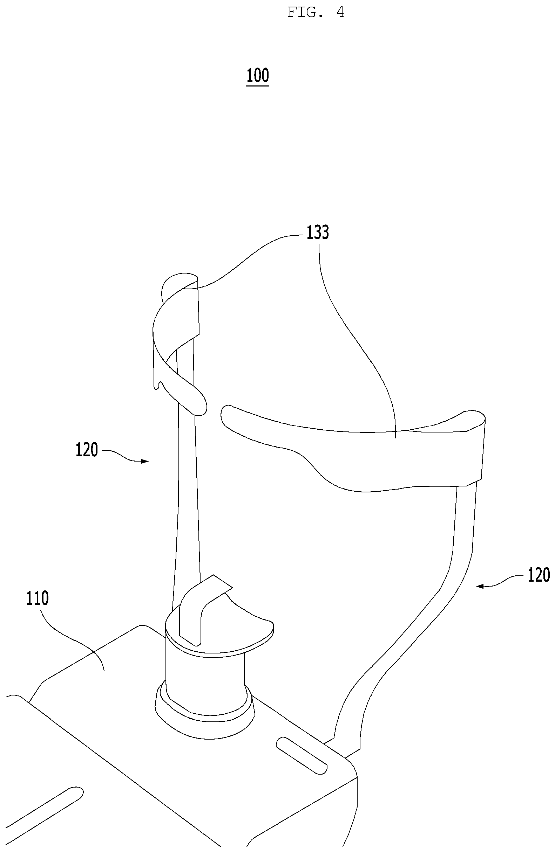

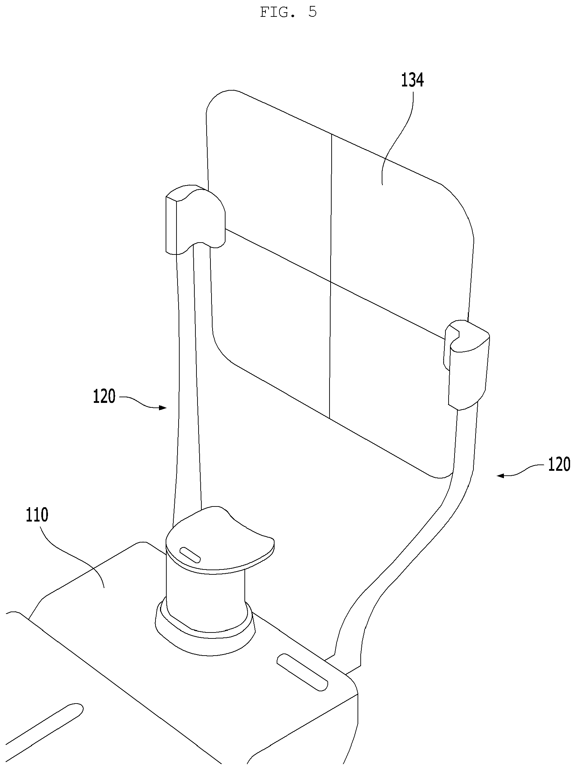

Hereinafter, the plurality of types of guide units 130 detachably provided to the guide structure 122 or the upper end part 121b or both of the device will be described. FIG. 2 is a schematic diagram illustrating a support unit 131, which is an example of a first guide unit of a subject alignment device according to an embodiment of the present disclosure. FIG. 3 is a schematic diagram illustrating a plug unit 132, which is an example of a second guide unit of a subject alignment device according to an embodiment of the present disclosure. FIG. 4 is a schematic diagram illustrating a face guide unit 133, which is another example of a first guide unit of a subject alignment device according to an embodiment of the present disclosure. FIG. 5 is a schematic diagram illustrating a plate unit 134, which is an example of a third guide unit of a subject alignment device according to an embodiment of the present disclosure.

A plurality of types of guide units 130 may include at least one selected from the group of a pair of support units 131, a pair of plug units 132, a face guide unit 133, and a plate unit 134. The pair of support units 131 come into close contact with the left and right temples of the target being radiographed. The pair of plug units 132 are respectively inserted into the left and right ears of the target being radiographed. The face guide unit 133 comes into close contact with the face of the target being radiographed. The plate unit 134 comes into close contact with one side of the target being radiographed.

Referring to FIG. 2, the support units 131 may be provided in a shape having a curved surface that bends in the direction in which the target being radiographed is positioned, so as to come into close contact with the left and right temples of a typical adult. Through such a curved-surface shape, the head is fixed while the left and right temples of the target being radiographed are in close contact with the support units, thereby enabling accurate radiography. In addition, referring to FIG. 2, the support units 131 may be mounted to the upper end parts 121a. The support units 131 may be temple supports and may be used for CT radiography, but are not limited thereto.

Referring to FIG. 3, the plug unit 132 may be provided in a shape having a protrusion that protrudes toward the direction in which the target being radiographed is positioned, so as to be inserted into the left and right ear canals of the head. Through this, the protrusions are inserted into the ear canals of the target being radiographed during radiography, thereby fixing the head for accurate radiography. Herein, it is preferable that the plug units 132 maintain the same height, and each of the plug units 132 may be coupled in close contact with the guide structure 122. Referring to FIG. 3, the plug units 132 may be mounted to the guide structures 122. The plug units 132 may be, for example, ear rods, and may be used for cephalometric X-ray radiography, but are not limited thereto.

Referring to FIG. 4, the face guide unit 133 may be provided in a curved shape that is convex forward so as to come into in close contact with the front portion and the left and right side portions of the forehead. Through this, during radiography, the forehead of the target being radiographed comes into close contact with the rear surface of the face guide unit 133, thereby fixing the head for accurate radiography. Referring to FIG. 4, the face guide unit 133 may be mounted to the upper end part 121a. The face guide unit 133 may be used for CT radiography, but is not limited thereto.

Referring to FIG. 5, the plate unit 134 may include a plate member that is placed such that a normal of its surface faces toward the front or the rear of the device 100. For example, a coupling member may be placed to fix the plate member to the upper end part 121a or the guide structure 122 or both of the guide parts 120 in the pair, and the coupling member enables the plate member to be placed in a direction perpendicular to the front direction such that a portion (for example, a hand) of the target being radiographed comes into contact with one surface of the plate member. Through this, the target being radiographed is fixed and accurate radiography is achieved. In addition, the plate unit 134 may be used for carpus radiography, in which a hand is subjected to X-ray radiography, but is not limited thereto.

In addition, the present disclosure may provide an X-ray radiography apparatus (hereinafter, referred to as "the radiography apparatus") including the subject alignment device (the device) 100 according to the above-described embodiment of the present disclosure.

The radiography apparatus may be an apparatus for performing X-ray radiography on a target, such as a person, being radiographed. X-ray radiography may be CT radiography, cephalometric X-ray radiography, or carpus X-ray radiography, but is not limited thereto. In addition, the radiography apparatus may be, for example, an apparatus used for radiographing a human body in hospitals or dental clinics. That is, the device 100 may be a device for fixing or aligning the position or the posture or both of a part of the human body.

The above description of the present disclosure is for illustrative purposes only, and those skilled in the art to which the present disclosure pertains will understand that the present disclosure can be embodied in other specific forms without changing the technical ideas or essential characteristics of the present disclosure. Therefore, it should be understood that the embodiments described above are illustrative in all aspects and not restrictive. For example, each element described in a singular form can also be implemented in a distributed manner. Likewise, elements described as being distributed can be implemented in a combined form.

The scope of the present disclosure is defined by the appended claims rather than by the detailed description above. It shall be understood that all alterations or modifications derived from the meaning and scope of the claims and their equivalents are included in the scope of the present disclosure.

## Claims

1. A subject alignment device of an X-ray radiography apparatus for obtaining at least two types of X-ray images of a subject, the subject alignment device comprising:
a guide part(120) configured to guide the subject into a radiography position for obtaining the X-ray images; and
guide units(130) configured to be replaceably and detachably coupled to the guide part(120) according to the types of the X-ray images, and
**characterized in that** the guide units(130) contact different positions of the subject respectively to align the subject in a photographing posture for obtaining the X-ray image.

2. The subject alignment device of claim 1, wherein the guide part(120) include a pair of bars, placed facing each other with the radiography positions therebetween, and
the guide units(130) are configured to be replaceably and detachably coupled to the guide parts(120) in the pair, respectively.

3. The subject alignment device of claim 2, further comprising a guide structure(122) provided at each of the pair of bars and configured to determine mounting positions of the guide units(130).

4. The subject alignment device of claim 1, wherein the subject is a head,
the types of the X-ray images include a CT image, and
the guide units(130) include a first guide unit configured to come into close contact with the subject's temple or face for obtaining the CT image.

5. The subject alignment device of claim 1, wherein the subject is a head,
the types of the X-ray images include a cephalometric X-ray image of the head, and
the guide units(130) include a second guide unit configured to be inserted into the subject's ear for obtaining the cephalometric X-ray image.

6. The subject alignment device of claim 2, wherein the subject is a hand,
the types of the X-ray images include a carpus X-ray image of the hand, and
the guide units(130) include a third guide unit having a plate form and configured to come into close contact with the hand for obtaining the carpus X-ray image.

7. An X-ray radiography apparatus comprising a subject alignment device according to claim 1.
